(19) Europäisches Patentamt
European Patent Office
Office européen des brevets

(11) **EP 1 048 625 B1**

(12) **EUROPEAN PATENT SPECIFICATION**

(45) Date of publication and mention
of the grant of the patent:
**02.01.2004 Bulletin 2004/01**

(51) Int Cl.$^7$: **C03C 13/00**, C03C 13/06

(21) Application number: **00201380.3**

(22) Date of filing: **18.04.2000**

(54) **Biosoluble composition of glass fibres for the production of glass wools and the like**

Biolösliche Glasfaser-Zusammensetzung für die Herstellung von Glaswollen und dergleichen

Composition biosoluble de fibres de verre pour la production de laines de verre et similaires

(84) Designated Contracting States:
**AT BE CH CY DE DK ES FI FR GB GR IE IT LI LU
MC NL PT SE**
Designated Extension States:
**SI**

(30) Priority: **30.04.1999 ES 9900960
22.03.2000 ES 200000685**

(43) Date of publication of application:
**02.11.2000 Bulletin 2000/44**

(73) Proprietor: **Poliglas, S.A.**
**08210 Barbera del Valles, Barcelona (ES)**

(72) Inventors:
• **Mesa Sanchez, Manuel**
**43810 El Pla de Santa Maria (ES)**
• **Giro Guasch, Enrique**
**43810 El Pla de Santa Maria (ES)**

• **Pasalaigua Huguet, Jorge**
**43810 El Pla de Santa Maria (ES)**

(74) Representative: **Revenga Santos, Josep Luis**
**c/o Aguilar & Revenga**
**C. Consell de Cent 415, 5.o-1.a**
**08009 Barcelona (ES)**

(56) References cited:
**WO-A-98/43923          FR-A- 2 781 788
US-A- 5 055 428          US-A- 5 108 957**

• **POTTER R M ET AL: "GLASS FIBER
DISSOLUTION IN A PHYSIOLOGICAL SALINE
SOLUTION" GLASTECHNISCHE
BERICHTE,DE,VERLAG DER DEUTSCHEN
GLASTECHNISCHEN GESELLSCHAFT.
FRANKFURT, vol. 64, no. 1, 1991, pages 16-28,
XP000178832**

## Description

### Technical field to which the invention applies

The present invention refers to biosoluble (or biodegradable) compositions of glass fibres, according to claim 1, for the production of glass wools and the like.

### Prior art

In the last years, it has been raised that the particles released during the manufacture, storage and use of glass wool products and inhaled by individuals in their environment are potentially cancerogenic if gathered in the lung. Because of this, several standards and recommendations have been introduced to ensure that the fibres with which said glass wools are produced are bioremovable either by macrophage digestion and mucolytic expulsion or by dissolution in the lung body fluids.

In order to measure biosolubility of the particles capable of being inhaled, *in vivo* and *in vitro* tests are used.

*In vivo* biosolubility tests attempt to determine the average residence time of particles in the lung by means of different protocols, among which the *"Biopersistence of fibres. Short-term exposure by inhalation"*, emanating from the European Community (EC/TM/26 rev. 7, 1998). Its object is to calculate the half-life of the particles in the lung, by obtaining a numerical value showing the above mentioned half-life ($T_{1/2}$) in days.

*In vitro* tests are carried out by simulating the action of lung fluid over fibre particles to establish again a numerical value correlating to its presumed half-life ($T_{1/2}$) in the lung.

It is assumed that a fibre is acceptably biosoluble if its half-life is $\leq$ 10 days in an *in vivo* inhalation test.

Apart from fibre biosolubility tests *in vivo* and *in vitro*, and presumably upon considering that there does not exist an evident and irrefutable correlation between cancer and the gathering of said particles in the lung, the Authorities in Germany introduced (on 12 June 1998) a decree in which the so-called "KI index" ("*Dritte Verordnung zur Änderung der Gefahrstoffverordnung*") is established. This index is defined by the following formula:

$$KI = \%Na_2O + \%K_2O + \%CaO + \%MgO + \%B_2O_3 + \%BaO - 2*\%Al_2O_3.$$

With KI $\geq$ 40, a fibre is considered harmless, without the need to perform confirmatory tests. With KI < 40, it may be necessary to confirm the harmless nature of the fibre through biosolubility tests.

The prior art documents which are considered to be most representative to the present invention are the following: EP 0412878 --proposing that the fibre includes more than 0.1 % in weight of phosphorus pentoxide ($P_2O_5$) when the percentage in weight of alumina ($Al_2O_3$) is equal to or higher than 1 %--, EP 0738692 and EP 0738693 -- proposing high contents of boron oxide ($B_2O_3$) with the possible use of $P_2O_5$--, WO 9634836 --proposing fibres with KI $\geq$ 40--, FR 2758322 --proposing fibres with KI > 30, preferably KI > 35, and low ratios $R_2O/RO$ (ratio of alkaline oxides/alkaline-earth oxides)--, EP 0872458 and WO 9906332 --proposing relatively low contents of silica ($SiO_2$) and high KI.

The general trend reflected by the majority of the most recent prior art documents is that manufacturers have tried to increase the dissolution capacity of the fibre by decreasing the conventional amount of $SiO_2$ and $Al_2O_3$ and by the addition of new oxides, particularly $P_2O_5$ and sulphur trioxide ($SO_3$), also attempting to reach KI values $\geq$ 40.

However, the approach of the above mentioned trend has obvious disadvantages:

- the decrease of $SiO_2$ brings about a decrease in the melt viscosity;
- the decrease of $Al_2O_3$ implies a corresponding decrease in the mechanical strength of the fibres;
- the presence of $P_2O_5$ has a negative effect on the fibre strength to mechanical stresses, increases the production of dust and causes that the refractory of the furnaces (especially gas-fired furnaces) for manufacturing the fibre suffers the aggressive attack of $P_2O_5$;
- the presence of $SO_3$ determines a greater corrosion of the discs which produce the fibres.

With respect to the negative effect of $P_2O_5$ to the mechanical strength of the fibres, the inventors found through testing that an increase from 0 to 0.8 % of $P_2O_5$ in detriment, for example, of the same amount of $Al_2O_3$, results in a decrease nearly linear to the mechanical strength to 60 % of the original one.

### Disclosure of the invention

The present inventors assigned themselves the task of obtaining new biosoluble wool fibres. Unlike the current manufacturer's trend mentioned above, the experiments of the present inventors were carried out under the following general guidelines:

- firstly, the use of $P_2O_5$ and $SO_3$ was omitted;
- secondly, relatively high contents of $SiO_2$ and $Al_2O_3$ were maintained in order to enhance the viscosity of the melt and the mechanical strength of the fibres;
- thirdly, the amount of RO (sum of oxides of elements pertaining to column 2A of the Periodic Table, and in practice sum %CaO+%MgO) was decreased to improve the workability of the melt, and to avoid its devitrification during the fibre formation; and
- fourthly, the amount of $R_2O$ (sum of oxides of elements pertaining to column 1A of the Periodic Table, and in practice sum %Na$_2$O+%K$_2$O) was increased so as to compensate, from the biosolubility viewpoint, for the relatively high content of $Al_2O_3$.

Surprisingly and unexpectedly, the inventors were able to confirm through relevant testing, the biosolubility of the fibres of the invention, in spite of the fact that these fibres have a KI value well below 40 and even below 30. They also confirmed that the fibres of the invention imply a good compromise between, on the one hand, their biosolubility and, on the other hand, their mechanical properties and workability.

Thereby, the invention refers to a biosoluble composition of glass fibres for the production of glass wools and the like, according to claim 1.

According to an optional feature of the invention, in the composition

| %Na$_2$O+%K$_2$O) | 18.5-19.5 |
|---|---|
| %CaO+%MgO | 8-10. |

According to another optional feature of the invention, the composition satisfies the following equation:

$$\%Na_2O+\%K_2O+\%CaO+\%MgO+\%B_2O_3+\%BaO-2*\%Al_2O_3 \leq 33.$$

According to another optional feature of the invention, the composition satisfies the following equation:

$$\%Na_2O+\%K_2O+\%CaO+\%MgO+\%B_2O_3+\%BaO-2*\%Al_2O_3 \leq 32.$$

According to another optional feature of the invention, the composition has a maximum liquidus temperature (viscosity = $10^{2.5}$ poise) comprised between 1.050 and 1.170 °C, a drop temperature (viscosity = $10^3$ poise) comprised between 950 and 1.070 °C and a devitrification temperature comprised between 650 and 850 °C.

According to another optional feature of the invention, the composition has a maximum liquidus temperature (viscosity = $10^{2.5}$ poise) comprised between 1.050 and 1.150 °C, a drop temperature (viscosity = $10^3$ poise) comprised between 950 and 1.050 °C and a devitrification temperature comprised between 700 and 780 °C.

According to another optional feature of the invention, the composition is one of the following:

- $SiO_2$ 64.29 %; $Na_2O$ 18.12 %; $K_2O$ 0.47 %; CaO 6.07 %; MgO 2.97 %; $B_2O_3$ 5.95 %; and $Al_2O_3$ 1.85 % ($C_6$);

- $SiO_2$ 64.37 %; $Na_2O$ 18.46 %; $K_2O$ 0.34 %; CaO 5.68 %; MgO 2.95 %; $B_2O_3$ 6.01 %; and $Al_2O_3$ 1.95 % ($C_7$);

- $SiO_2$ 64.28 %; $Na_2O$ 19.02 %; $K_2O$ 0.41 %; CaO 5.34 %; MgO 2.72 %; $B_2O_3$ 5.91 %; and $Al_2O_3$ 2.11 % ($C_8$);

- $SiO_2$ 66.3 %; $Na_2O$ 17.6 %; $K_2O$ 0.52 %; CaO 5.95 %; MgO 2.82 %; $B_2O_3$ 4.8 %; and $Al_2O_3$ 1.75 % ($C_9$);

- $SiO_2$ 64.86 %; $Na_2O$ 18.06 %; $K_2O$ 0.51 %; CaO 5.94 %; MgO 2.94 %; $B_2O_3$ 5.52 %; and $Al_2O_3$ 2.02 % ($C_{10}$);

- $SiO_2$ 63.24 %; $Na_2O$ 17.94 %; $K_2O$ 0.38 %; CaO 6.01 %; MgO 3.02 %; $B_2O_3$ 6.74 %; and $Al_2O_3$ 2.48 % ($C_{11}$);

- $SiO_2$ 63.05 %; $Na_2O$ 19.41 %; $K_2O$ 0.44 %; CaO 6 %; MgO 2.96 %; $B_2O_3$ 5.49 %; and $Al_2O_3$ 2.51 % ($C_{12}$);

- $SiO_2$ 62.28 %; $Na_2O$ 20.11 %; $K_2O$ 0.41 %; CaO 5.97 %; MgO 2.76 %; $B_2O_3$ 5.41 %; and $Al_2O_3$ 2.8 % ($C_{13}$);

further including up to about 2 % in weight of impurities and other conventional compounds such as $Fe_2O_3$, but $P_2O_5$ and $SO_3$ being excluded from all of said compositions.

The scope of the invention also embraces the glass wool products that include fibres having the above mentioned compositions.

## Brief description of the drawings

In the enclosed sheet of drawings:

Fig. 1 is a very schematic representation of a test apparatus used for calculating the dissolution rate constant, $K_{SiO2}$, of the Compositions $C_6$ to $C_{13}$ according to the invention;

Fig. 2 is a graph where the dissolution rate constant $K_{SiO2}$ (in ng of $SiO_2$*$cm^{-2}$*$h^{-1}$) of each one of the Compositions $C_6$ to $C_{13}$ according to the invention are represented, as a function of the analysis time, in hours; and

Fig. 3 is a graph where the behaviour of the dissolution rate constant $K_{SiO2}$ of each of the Compositions $C_7$ to $C_{13}$ according to the invention, with respect to the dissolution rate constant of Composition $C_6$, is represented, as a function of the analysis time, in hours.

The invention will be further disclosed by means of the Reference Compositions $C_1$ to $C_5$ and the Compositions $C_6$ to $C_{13}$, which represent Examples of the invention and whose formulations and test results are shown in Table I.

## Preliminary tests

Preliminary tests were carried out, with and without the use of $P_2O_5$ and $SO_3$. The following Reference Compositions $C_1$ to $C_5$ were chosen because of their significance.

## Reference Compositions $C_1$ to $C_3$

In a first series of tests, the Reference Compositions $C_1$, $C_2$ and $C_3$ (see Table I) are prepared. In all of them $P_2O_5$ is included, and a relatively high content of $R_2O$ ($\geq$ 17.65 %) and a relatively low content of RO ($\leq$ 10.45 %) are maintained, while variable and conventional amounts of $B_2O_3$, $SO_3$ and ferric oxide ($Fe_2O_3$) are used. In Reference Composition $C_1$, the $Al_2O_3$ content is about twice as much as in the Reference Compositions $C_2$ and $C_3$.

It can be seen in Table I (row $T_{1/2}$) that by maintaining a relatively high $SiO_2$ content, in the Reference Compositions $C_1$, $C_2$ and $C_3$ aforementioned:

1. Decrease of $Al_2O_3$ percentage enhances biosolubility.
2. Increase of $P_2O_5$ and $SO_3$ enhances biosolubility.
3. Maintaining of a high content of $R_2O$ enhances biosolubility.

The mechanical strength of the fibres (especially, in Reference Compositions $C_2$ and $C_3$) was remarkably lower than the strength of fibres obtained with conventional non-biosoluble compositions.

## Reference Compositions $C_4$ and $C_5$

Two other biosoluble Reference Compositions $C_4$ and $C_5$ are prepared. In Reference Composition $C_4$ a relatively high content of $Al_2O_3$ (2.32 %), a relatively low content of $R_2O$ (15.69 %) and a relatively high content of RO (10.8 %) are used, and in both of them conventional amounts of $Fe_2O_3$ are included. In view of the relatively high $Al_2O_3$ content, the $B_2O_3$ content is increased proportionally to improve biosolubility without impairing workability. In Reference Composition $C_5$ a relatively low content of $Al_2O_3$ (1.72 %), a relatively high content of $R_2O$ (17.53 %) and relatively low content of RO (10.12 %) are used. In neither of these Reference Compositions $P_2O_5$ or $SO_3$ is included. In Table I the test results can be seen, leading to the following conclusions:

4. Biosoluble glasses can be obtained without $P_2O_5$ or $SO_3$.
5. The suitability of maintaining the sum of alkali oxides high, with the corresponding decrease of alkali-earth oxides, is confirmed.

The mechanical strength of the fibres of Reference Compositions $C_4$ and $C_5$ was much higher than the strength of fibres obtained with the Reference Compositions $C_1$ to $C_3$.

## Preferred embodiments of carrying out the invention

Going on from the above conclusions, in a new series of tests, Compositions $C_6$ to $C_{13}$ according to the invention are prepared. In all of them, the $R_2O$ content is increased (in detriment of RO), and the $Al_2O_3$ content is also increased. Of course, according to the above mentioned guidelines $P_2O_5$ and $SO_3$ are excluded.

## Compositions $C_6$ to $C_8$ according to the invention

The change in chemical composition resides in a progressive increase in $Al_2O_3$ (1.85 %, 1.95 % and 2.11 %). On the other hand, this increase is tried to be balanced by a corresponding increase of $Na_2O$ (18.12 %, 18.46 % and 19.02 %) in detriment of a progressive decrease of RO. The dissolution rate constant, $K_{SiO2}$, clearly decreases from $C_6$ to $C_8$. The amount of $Na_2O$ added is not sufficient to counteract the effect of $Al_2O_3$ in the stabilisation of the crystal network. The decrease of RO fails to counteract this effect of the increase in $Al_2O_3$.

## Compositions $C_9$ to $C_{11}$ according to the invention

The strategy pursued has been to gradually increase the $Al_2O_3$ content (1.75 %, 2.02 % and 2.48 %), increasing at the same time the amount of $B_2O_3$ (4.80 %, 5.52 % and 6.74 %). The analysis of the dissolution rate constant, $K_{SiO2}$, shows a decreased value provoked by the increase in $Al_2O_3$. The $B_2O_3$ added is not able to totally balance the increase of $Al_2O_3$ produced from $C_9$ to $C_{11}$.

However, the values of the dissolution rate constants, $K_{SiO2}$, for $C_{10}$ and $C_{11}$ are higher than for $C_8$ which means that in an *in vivo* biosolubility test the result would be favourable.

The constant $K_{SiO2}$ obtained for $C_9$ is consistent with that obtained for $C_6$. The constant of $C_6$ is slightly more favourable because $C_6$ contains a little more $B_2O_3$.

## Compositions $C_{12}$ and $C_{13}$ according to the invention

In these latter compositions exemplifying the invention the content of $Al_2O_3$ and of $Na_2O$ is extremely high, while the $B_2O_3$ content is relatively low. The dissolution rate constants, $K_{SiO2}$, are consistent with each other and determined by the $Al_2O_3/Na_2O$ ratio. Composition $C_{12}$ would be biosoluble in an *in vivo* test because its dissolution rate constant value is higher than that obtained in Composition $C_8$.

## Discussion of results in Table I:

In Table I the formulations and the test results of Reference Compositions $C_1$ to $C_5$ and Compositions $C_6$ to $C_{13}$ according to the invention are shown. These results allow to deduce that:

1. The $Al_2O_3$ effect clearly impairs the biosolubility;
2. Elements which most enhance biosolubility are $Na_2O$, which enhances the dissolution of the vitreous network, and $B_2O_3$, which does likewise but in a more moderate extension;
3. Although glasses of Compositions $C_6$ to $C_{13}$ according to the invention have a KI value close to and even lower than 30, its estimated $T_{1/2}$ value and/or its biosolubility value are very favourable;
4. Compositions $C_6$ to $C_{13}$ of the invention provide temperature ranges for fibre production ($F_{\cdot fibr}$) well adapted to current manufacture methods and apparatuses;
5. Compositions $C_6$ to $C_{13}$ of the invention present devitrification temperatures ($Temp_{\cdot dev}$) more favourable than Reference Compositions.

Mechanical strength values of Compositions $C_6$ to $C_{13}$ according to the invention were much higher than the respective values of Reference Compositions $C_1$ to $C_5$.

## Table I

| | Reference Compositions | | | | | Compositions according to the invention | | | | | | | |
|---|---|---|---|---|---|---|---|---|---|---|---|---|---|
| | $C_1$ | $C_2$ | $C_3$ | $C_4$ | $C_5$ | $C_6$ | $C_7$ | $C_8$ | $C_9$ | $C_{10}$ | $C_{11}$ | $C_{12}$ | $C_{13}$ |
| $SiO_2$ | 63.98 | 64.6 | 65.8 | 65.1 | 64.6 | 64.29 | 64.37 | 64.28 | 66.3 | 64.86 | 63.24 | 63.05 | 62.28 |
| $Na_2O$ | 17.8 | 17 | 17.3 | 14.4 | 16.6 | 18.12 | 18.46 | 19.02 | 17.6 | 18.06 | 17.94 | 19.41 | 20.11 |
| $K_2O$ | 0.43 | 0.65 | 0.55 | 1.29 | 0.93 | 0.47 | 0.34 | 0.41 | 0.52 | 0.51 | 0.38 | 0.44 | 0.41 |
| $CaO$ | 7.8 | 7.7 | 7.35 | 7.13 | 6.6 | 6.07 | 5.68 | 5.34 | 5.95 | 5.94 | 6.01 | 6 | 5.97 |
| $MgO$ | 2.65 | 2.55 | 2.4 | 3.67 | 3.52 | 2.97 | 2.95 | 2.72 | 2.82 | 2.94 | 3.02 | 2.96 | 2.76 |
| $B_2O_3$ | 4.2 | 5.8 | 4.2 | 6 | 5.93 | 5.95 | 6.01 | 5.91 | 4.80 | 5.52 | 6.74 | 5.49 | 5.41 |
| $P_2O_5$ | 0.81 | 0.2 | 1 | 0 | 0 | 0 | 0 | 0 | 0 | 0 | 0 | 0 | 0 |
| $SO_3$ | 0.32 | 0.41 | 0.41 | 0 | 0 | 0 | 0 | 0 | 0 | 0 | 0 | 0 | 0 |
| $Fe_2O_3$ | 0.12 | 0.11 | 0.11 | 0.11 | 0.09 | 0.28 | 0.24 | 0.21 | 0.26 | 0.15 | 0.19 | 0.14 | 0.26 |
| $Al_2O_3$ | 1.95 | 1 | 0.9 | 2.32 | 1.72 | 1.85 | 1.95 | 2.11 | 1.75 | 2.02 | 2.48 | 2.51 | 2.8 |
| $R_2O$ | 18.23 | 17.65 | 17.85 | 15.69 | 17.53 | 18.59 | 18.8 | 19.43 | 18.12 | 18.57 | 18.32 | 19.85 | 20.52 |
| $RO$ | 10.45 | 10.25 | 9.75 | 10.80 | 10.12 | 9.04 | 8.63 | 8.06 | 8.77 | 8.88 | 9.03 | 8.96 | 8.73 |
| $R_2O/RO$ | 1.74 | 1.72 | 1.83 | 1.45 | 1.73 | 2.06 | 2.18 | 2.41 | 2.07 | 2.09 | 2.03 | 2.22 | 2.35 |
| $Temp._{liq}$ | 1115.0 | 1091.5 | 1134.0 | 1146.4 | 1114.0 | 1103.6 | 1097.9 | 1095.2 | 1140.37 | 1118.13 | 1077.81 | 1076 | 1066.6 |
| $Temp._{drop}$ | 1019.4 | 1001.2 | 1037.5 | 1049.0 | 1016.4 | 1006.1 | 1000.1 | 996.6 | 1038.37 | 1018.89 | 983.74 | 978.02 | 968.81 |
| $Temp._{dev}$ | 827.2 | 803.7 | 809.0 | 888.4 | 796.9 | 753.5 | 731.4 | 708.4 | 772.82 | 765.85 | 753.35 | 716.07 | 706.6 |
| $F._{fibr}$ | 192.20 | 197.50 | 228.50 | 160.60 | 219.50 | 252.6 | 268.7 | 288.2 | 265.55 | 253.04 | 230.39 | 261.95 | 262.21 |
| $KI$ | 28.98 | 31.70 | 30.00 | 27.85 | 30.14 | 29.88 | 29.54 | 29.18 | 28.19 | 28.93 | 29.13 | 29.28 | 29.06 |
| $T_{1/2}$ | 10.4 | 5.9 | 5 | 10.1 | 6.1 | 6.4 | 7 | 8.5 | -- | -- | -- | -- | -- |
| $K_{SiO_2}$ (t=1 day) | -- | -- | -- | -- | -- | 159.4 | 147.3 | 114.7 | 162.65 | 119.3 | 117 | 136 | 111.8 |
| $K_{SiO_2}$ (t=2 days) | -- | -- | -- | -- | -- | 137.6 | 132 | 97.1 | 127.1 | 106.2 | 103.6 | 114.8 | 93.2 |
| $K_{SiO_2}$ (t=3 days) | -- | -- | -- | -- | -- | 135.3 | 123.4 | 87.1 | 116 | 97 | 94.8 | 106 | 80.4 |
| $K_{SiO_2}$ (t=4 days) | -- | -- | -- | -- | -- | 135.1 | 118.7 | 81.4 | 112.5 | 90.51 | 90.0 | 102.3 | 71.5 |

EP 1 048 625 B1

In Table I:

| | | |
|---|---|---|
| RO | = | Sum of oxides of elements pertaining to column 2A of the Periodic Table |
| $R_2O$ | = | Sum of oxides of elements pertaining to column 1A of the Periodic Table |
| Temp.$_{liq.}$ | = | Maximum liquidus temperature (viscosity = $10^{2.5}$ poise) |
| Temp.$_{drop}$ | = | Reference temperature for glass fiberisation or workability (viscosity = $10^3$ poise) |
| Temp.$_{dev}$ | = | Devitrification or crystal-growth temperature |
| F.$_{fibr.}$ | = | Difference between Temp.$_{drop}$ and Temp.$_{dev.}$. It is an indication of temperature ranges where fibre formation is possible with usual methods and apparatuses |
| $T_{1/2}$ | = | Half-life or time of elimination from the lung, in days, of 50 % of particles longer than 20 micron |
| $K_{SiO2}$ | = | $SiO_2$ dissolution rate constant which will be discussed below in more detail; t = test days. |

**Calculation of glass dissolution rate constant, $K_{glass}$**

**Conventional methodology:**

The calculation of glass dissolution rate constant, $K_{glass}$, in different fluids is known; one of the most interesting tests in the mineral wool field is the one in which a glass composition is attacked with a fluid simulating, in terms of compounds and concentrations, lung fluid. This simulated biological fluid is also known as "Gamble's solution" and its composition is the following:

| | |
|---|---|
| NaCl | 6.415 g/l |
| $NaHCO_3$ | 32.70 g/l |
| CaCl | 20.19 g/l |
| $Na_2HPO_4.12H_2O$ | 0.358 g/l |
| $Na_2SO_4$ | 0.079 g/l |
| $MgCl_2.6H_2O$ | 0.212 g/l |
| Glycine | 0.118 g/l |
| $Na_x$ Citrate+tartrate+lactate+pyruvate | 0.680 g/l |
| Formaldehyde | 1.000 ml/l |

In the conventional methodology, some variations of this fluid are also used.
In prior art, this test has been carried out on glass fibres having a well controlled distribution of diameter and length, in order to calculate the specific surface in the most precise way possible. The test implies a separation and characterization step of the fibres. This is a long, tedious and expensive procedure and requires a sophisticated instrumentation.
The test is carried out controlling three instrumental parameters: pH, temperature and flow rate.
The balances of dissolution reactions in the test are the following:

$$(Si\text{-}O\text{-}X)_{glass} + H_2O \leftrightarrow (Si\text{-}OH)_{glass} + X^+ + OH^-$$

$$(Si\text{-}O\text{-}X)_{glass} + H^+ \leftrightarrow (Si\text{-}OH)_{glass} + X^+$$

$$(Si\text{-}O\text{-}X)_{glass} + OH^- \leftrightarrow (Si\text{-}OH)_{glass} + (Si\text{-}O\text{-})_{glass}$$

$$(Si\text{-}O\text{-}X)_{glass} + OH^- \leftrightarrow (Si\text{-}OH)_{dissolution} + (Si\text{-}O\text{-})_{glass} \text{ (glass network dissolution).}$$

These reactions are produced at a surface level and usually the dissolution constant $K_{glass}$ is expressed as nanograms of glass dissolved per square centimetre and hour (ng glass*cm$^{-2}$*h$^{-1}$).

**Methodology used for the calculation of the dissolution rate constant $K_{SiO2}$ of $SiO_2$ in the Compositions $C_6$ to $C_{13}$ according to the invention:**

Using the aforementioned conventional methodology but modified as said below, tests have been carried out to estimate the $SiO_2$ dissolution rate constant, $K_{SiO2}$, in order to observe its evolution as glass composition is changed.
Starting from the fact that the half-lives in the lung or *in vivo* of Compositions $C_6$ to $C_8$ according to the invention are known (see values of row "$T_{1/2}$" in Table I):

- Composition $C_5$ showed to be biosoluble ($T_{1/2}$ = 6.4) by means of an inhalation *in vivo* test, since the half-life was remarkably lower than 10 days (Directive 97/69/CE). The value of the dissolution rate constant, with t = 4 days, for this composition is 135.1 ng $SiO_2$*cm$^{-2}$*h$^{-1}$. This value is used as the highest reference value.
- Composition $C_8$ showed to be the most unfavourable one ($T_{1/2}$ = 8.5) as far as the half-life period, by inhalation, is concerned. The value of the dissolution rate constant, with t = 4 days, for this Composition $C_8$ is 81.4 ng $SiO_2$*cm$^{-2}$*h$^{-1}$ and is used as the lowest reference value.

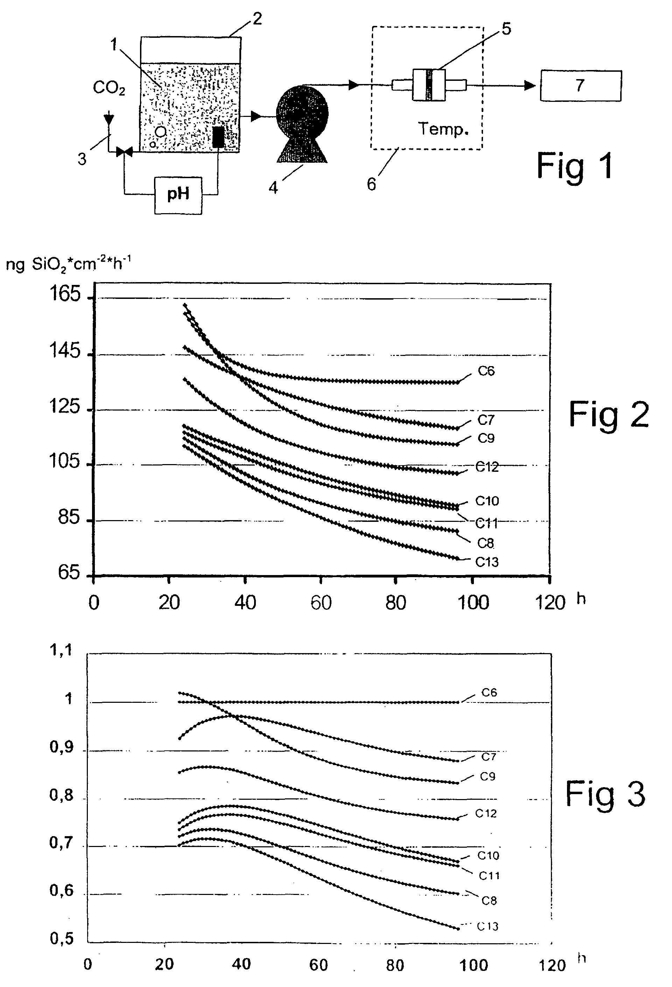

Although a good correlation between *in vitro* dissolution constant and the period of half-life has not been proved, said constant can serve as an indication when the real chemical behaviour in the lungs of a living being is evaluated.
The conventional methodology to calculate the dissolution constant, $K_{glass}$, in Gamble's solution, has been modified by the inventors to achieve a quicker and simpler method allowing the comparison of different compositions, although not intended to establish values of dissolution constants directly comparable to those obtained through the conventional methodology used with fibres.
The main differences between both methodologies are the manner in which the sample is introduced into the test apparatus and the duration of the test.
In the methodology used here, a glass powder sample with a known extremely high (about 1 m$^2$/g) specific surface has been used in order to eliminate the problems that arise when --as happens in the conventional methodology-- the comparison between two compositions of fibres that have different diameters and lengths is needed. With the glass powder, it is possible to avoid the expensive step of the conventional methodology for separating and characterizing the fibres.
With the methodology used here, only the chemical attack of fluid on the glass powder is evaluated.
The tests are carried out in a relatively short period of time, from about 96 to 120 hours.
The test procedure (which does not form part of the invention) is described hereafter with reference to Fig. 1:
The fluid (Gamble's solution) 1, contained in a tank 2, is continuously corrected, in order to obtain a constant pH value, by means of $CO_2$ bubbling 3. A peristaltic pump 4 draws fluid to the sample cell 5, which contains the glass powder sample and which is maintained at a constant temperature (Temp.) with a thermostatic bath 6. The fluid portion with solved glass is collected in bottles at the outlet of the sample cell and analyzed by atomic absorption in 7.
The pH, temperature and flow rate values chosen for the tests of the glass compositions are the following:

| pH | 7.6 ± 0.1 |
| --- | --- |
| Temp. | 37 ± 0.5 °C |
| Flow rate | 140-180 ml/day. |

The test results are presented, partly, in the above Table I and, partly, in Figs. 2 and 3, described above.

It is understood that the manufacturers in this technical field experience problems in composition variation from one raw material batch to another one, which make admissible tolerances in ±2 % of absolute value, in some of the components of a given formulation. For this reason, in spite of the apparent precision with which the weight percentages of the invention compositions are given, the skilled in this art must understand that such values are nominal and that the claims also cover the compositions that respond to said nominal values affected by the usual tolerances in this technical field.

**Claims**

1.   A biosoluble composition of glass fibres for the production of glass wools and the like, **characterized in that** it includes the following compounds in the specified weight percentages of the whole composition:

| | |
|---|---|
| $\%SiO_2$ | 62 - 66.5 |
| $\%Al_2O_3$ | 1.5 - 2.9 |
| $\%Na_2O$ | $\geq 17.5$ |
| $\%K_2O$ | 0.1 - 1.0 |
| $\%Na_2O+\%K_2O$ | 17.9 - 21.1 |
| $\%CaO$ | 5.0 - 7.0 |
| $\%MgO$ | $2 \leq 3.5$ |
| $\%CaO+\%MgO$ | 7.95 - 9.25 |
| $\%R_2O/\%RO$ | > 1.65 |
| $\%B_2O_3$ | 4.7 - 7 |

where RO is the sum of the included oxides of elements pertaining to column 2A of the Periodic Table and $R_2O$ is the sum of the included oxides of elements pertaining to column 1A of the Periodic Table, the composition further including up to about 2 % in weight of impurities and other conventional compounds such as $Fe_2O_3$, but $P_2O_5$ and $SO_3$ being excluded from the composition.

2.   The composition according to claim 1, wherein:

| | |
|---|---|
| $\%Na_2O+\%K_2O$ | 18.5 - 19.5 |
| $\%CaO+\%MgO$ | 8 - 10 |

3.   The composition according of claim 1 or 2, **characterized in that** it satisfies the following equation:

$$\%Na_2O+\%K_2O+\%CaO+\%MgO+\%B_2O_3+\%BaO-2*\%Al_2O_3 \leq 33.$$

4.   The composition according to claim 1 or 2, **characterized in that** it satisfies the following equation:

$$\%Na_2O+\%K_2O+\%CaO+\%MgO+\%B_2O_3+\%BaO-2*\%Al_2O_3 \leq 32.$$

5.   The composition according to any of claims 1 to 4, **characterized in that** it has a maximum liquidus temperature (viscosity = $10^{2.5}$ poise) comprised between 1 050 and 1 170 °C, a drop temperature (viscosity = $10^3$ poise) comprised between 950 and 1 070 °C and a devitrification temperature comprised between 650 and 850 °C.

6.   The composition according to any of claims 1 to 4, **characterized in that** it has a maximum liquidus temperature (viscosity = $10^{2.5}$ poise) comprised between 1 050 and 1 150 °C, a drop temperature (viscosity = $10^3$ poise) comprised between 950 and 1 050 °C and a devitrification temperature comprised between 700 and 780 °C.

7.   The composition according to one of the claims 1 to 6, **characterized in that** it is one of the following:

   -    $SiO_2$ 64.29 %; $Na_2O$ 18.12 %; $K_2O$ 0.47 %; $CaO$ 6.07 %; $MgO$ 2.97 %; $B_2O_3$ 5.95 %; and $Al_2O_3$ 1.85 % ($C_6$);

- SiO$_2$ 64.37 %; Na$_2$O 18.46 %; K$_2$O 0.34 %; CaO 5.68 %; MgO 2.95 %; B$_2$O$_3$ 6.01 %; and Al$_2$O$_3$ 1.95 % (C$_7$);

- SiO$_2$ 64.28 %; Na$_2$O 19.02 %; K$_2$O 0.41 %; CaO 5.34 %; MgO 2.72 %; B$_2$O$_3$ 5.91 %; and Al$_2$O$_3$ 2.11 % (C$_8$);

**8.** The composition according to one of the claims 1, 3 to 6, **characterized in that** it is one of the following:

- SiO$_2$ 66.3 %; Na$_2$O 17.6 %; K$_2$O 0.52 %; CaO 5.95 %; MgO 2.82 %; B$_2$O$_3$ 4.8 %; and Al$_2$O$_3$ 1.75 % (Cg);

- SiO$_2$ 64.86 %; Na$_2$O 18.06 %; K$_2$O 0.51 %; CaO 5.94 %; MgO 2.94 %; B$_2$O$_3$ 5.52 %; and Al$_2$O$_3$ 2.02 % (C$_{10}$);

- SiO$_2$ 63.24 %; Na$_2$O 17.94 %; K$_2$O 0.38 %; CaO 6.01 %; MgO 3.02 %; B$_2$O$_3$ 6.74 %; and Al$_2$O$_3$ 2.48 % (C$_{11}$);

- SiO$_2$ 63.05 %; Na$_2$O 19.41 %; K$_2$O 0.44 %; CaO 6 %; MgO 2.96 %; B$_2$O$_3$ 5.49 %; and Al$_2$O$_3$ 2.51 % (C$_{12}$);

- SiO$_2$ 62.28 %; Na$_2$O 20.11 %; K$_2$O 0.41 %; CaO 5.97 %; MgO 2.76 %; B$_2$O$_3$ 5.41 %; and Al$_2$O$_3$ 2.8 % (C$_{13}$).

**9.** A glass wool product, wherein fibres of the composition defined in claims 1-8 are included.

**Patentansprüche**

**1.** Biolösliche Glasfaser-Zusammensetzung für die Herstellung von Glaswolle und dergleichen, **dadurch gekennzeichnet, daß** sie in spezifischen Gewichtsprozenten der gesamten Zusammensetzung die folgenden Mischungen umfassen:

| | |
|---|---|
| %SiO$_2$ | 62 - 66,5 |
| %Al$_2$O$_3$ | 1,5 - 2,9 |
| %Na$_2$O | $\geq$ 17,5 |
| %K$_2$O | 0,1 - 1,0 |
| %Na$_2$O + % K$_2$O | 17,9 - 21,1 |
| %CaO | 5,0 - 7,0 |
| %MgO | 2 $\leq$ 3,5 |
| %CaO + %MgO | 7,95 - 9,25 |
| %R$_2$O / %RO | > 1,65 |
| %B$_2$O$_3$ | 4,7 - 7 |

wobei RO die Summe der eingeschlossenen Oxide der Elemente sind, welche zur Spalte 2A des Perioden Systems gehören, R$_2$O die Summe der eingeschlossenen Oxide der Elemente sind, welche zu der Spalte 1A des Periodensystems gehören, und wobei ferner die Zusammensetzung einschließlich ungefähr 2 % des spezifischen Gewichtes von Verunreinigungen und anderen üblichen Zusammensetzungen wie Fe$_2$O$_3$ umfaßt, wobei jedoch P$_2$O$_5$ und SO$_3$ von der Zusammensetzung ausgeschlossen sind.

**2.** Zusammensetzung nach Anspruch 1 mit

| | |
|---|---|
| %Na$_2$O + % K$_2$O | 18,5 - 19,5 |
| %CaO + %MgO | 8 -10 |

**3.** Zusammensetzung nach Anspruch 1 oder 2, **dadurch gekennzeichnet, daß** sie der folgenden Gleichung entspricht:

$$\%Na_2O + \% K_2O + \%CaO + \%MgO + \%B_2O_3 + \%BaO - 2 \times \%Al_2 O_3 \leq 33.$$

**4.** Zusammensetzung nach Anspruch 1 oder 2, **dadurch gekennzeichnet, daß** sie der folgenden Gleichung entspricht:

$$\%Na_2O + \% K_2O + \%CaO + \%MgO + \%B_2O_3 + \%BaO - 2 \times \%Al_2O_3 \leq 32$$

**5.** Zusammensetzung nach einem der Ansprüche 1 bis 4, **dadurch gekennzeichnet, daß** sie eine maximale Verflüssigungstemperatur (Viskosität = $10^{2,5}$ poise) zwischen 1050 und 1 170°C, eine Tropftemperatur (Viskosität = $10^3$ poise) zwischen 950 und 1070°C und eine Verglasungstemperatur zwischen 650 und 850°C umfaßt.

**6.** Zusammensetzung nach einem der Ansprüche 1 bis 4, **dadurch gekennzeichnet, daß** sie eine maximale Verflüssigungstemperatur (Viskosität = $10^{2,5}$ poise) zwischen 1050 und 1 150°C , eine Tropftemperatur (Viskosität = $10^3$ poise) zwischen 950 und 1050°C und eine Verglasungstemperatur zwischen 700 und 780°C umfaßt.

**7.** Zusammensetzung nach einem der Ansprüche 1 bis 6 **gekennzeichnet durch** folgendes:

- $SiO_2$ 64,29%;$Na_2O$ 18,12%; $K_2O$ 0,47%; $CaO$ 6,07%; $MgO$ 2,97%; $B_2O_3$ 5,95%; und $Al_2O_3$ 1,85% ($C_6$);

- $SiO_2$ 64,37%;$Na_2O$ 18,46%; $K_2O$ 0,34%; $CaO$ 5,68%; $MgO$ 2,95%; $B_2O_3$ 6,01%; und $Al_2O_3$ 1,95% ($C_7$);

- $SiO_2$ 64,28%; $Na_2O$ 19,02%; $K_2O$ 0,41 %; $CaO$ 5,34%; $MgO$ 2,72%; $B_2O_3$ 5,91%; und $Al_2O_3$ 2,11% ($C_8$);

**8.** Zusammensetzung nach einem der Ansprüche 1, 3 bis 6 **gekennzeichnet durch** folgendes:

- $SiO_2$ 66,3%; $Na_2O$ 17,6%; $K_2O$ 0,52%; $CaO$ 5,95%; $MgO$ 2,82%; $B_2O_3$ 4,8%; and $Al_2O_3$ 1,75% ($C_9$);

- $SiO_2$ 64,86%; $Na_2O$ 18,06%; $K_2O$ 0,51%; $CaO$ 5,94%; $MgO$ 2,94%; $B_2O_3$ 5,52%; und $Al_2O_3$ 2,02% ($C_{10}$);

- $SiO_2$ 63,24%; $Na_2O$ 17,94%; $K_2O$ 0,38%; $CaO$ 6,01%; $MgO$ 3,02%; $B_2O_3$ 6,74%; und $Al_2O_3$ 2,48% ($C_{11}$);

- $SiO_2$ 63,05%; $Na_2O$ 19,41 %; $K_2O$ 0,44%; $CaO$ 6%; $MgO$ 2,96%; $B_2O_3$ 5,49%; und $Al_2O_3$ 2,51 % ($C_{12}$);

- $SiO_2$ 62,28%; $Na_2O$ 20,11 %; $K_2O$ 0,41 %; $CaO$ 5,97%; $MgO$ 2,76%; $B_2O_3$ 5,41%; und $Al_2O_3$ 2,8% ($C_{13}$);

**9.** Glaswolleprodukt umfassend Fasern der Zusammensetzung, wie in den Ansprüchen 1 bis 8 definiert.

## Revendications

**1.** Composition biosoluble de fibres de verre pour la production de laines de verre et analogues, **caractérisée en ce qu'**elle contient les composés suivants, dans les pourcentages pondéraux précisés par rapport à la composition totale :

| | |
|---|---|
| % $SiO_2$ | 62 - 66,5 |
| % $Al_2O_3$ | 1,5 - 2,9 |
| % $Na_2O$ | ≥ 17,5 |
| % $K_2O$ | 0,1 - 1,0 |
| % $Na_2O$ + % $K_2O$ | 17,9 - 21,1 |
| % $CaO$ | 5,0 - 7,0 |
| % $MgO$ | 2 ≤ 3,5 |
| % $CaO$ + % $MgO$ | 7,95 - 9,25 |
| % $R_2O$ / % $RO$ | > 1,65 |
| % $B_2O_3$ | 4,7 - 7 |

dans laquelle RO est la somme des oxydes contenus d'éléments appartenant à la colonne 2A du tableau périodique et $R_2O$ est la somme des oxydes contenus d'éléments appartenant à la colonne 1A du tableau périodique, la composition contenant en outre jusqu'à environ 2% en poids d'impuretés et d'autres composés classiques tels que $Fe_2O_3$, mais $P_2O_5$ et $SO_3$ étant exclus de la composition.

**2.** Composition selon la revendication 1, dans laquelle :

| % Na$_2$O + % K$_2$O | 18,5 - 19,5 |
|---|---|
| %CaO+%MgO | 8 -10 |

**3.** Composition selon la revendication 1 ou 2, **caractérisée en ce qu'**elle satisfait à l'équation suivante :

$$\% \text{ Na}_2\text{O} + \% \text{ K}_2\text{O} + \% \text{ CaO} + \% \text{ MgO} + \% \text{ B}_2\text{O}_3 + \% \text{ BaO} - 2 \times \% \text{ Al}_2\text{O}_3 \leq 33.$$

**4.** Composition selon la revendication 1 ou 2, **caractérisée en ce qu'**elle satisfait à l'équation suivante :

$$\% \text{ Na}_2\text{O} + \% \text{ K}_2\text{O} + \% \text{ CaO} + \% \text{ MgO} + \% \text{ B}_2\text{O}_3 + \% \text{ BaO} - 2 \times \% \text{ Al}_2\text{O}_3 \leq 32.$$

**5.** Composition selon l'une quelconque des revendications 1 à 4, **caractérisée en ce qu'**elle possède une température maximale de liquidus (viscosité = 10$^{2,5}$ poises) comprise entre 1 050 et 1 170°C, une température de goutte (viscosité = 10$^3$ poises) comprise entre 950 et 1 070°C, et une température de dévitrification comprise entre 650 et 850°C.

**6.** Composition selon l'une quelconque des revendications 1 à 4, **caractérisée en ce qu'**elle possède une température maximale de liquidus (viscosité = 10$^{2,5}$ poises) comprise entre 1 050 et 1 150°C, une température de goutte (viscosité = 10$^3$ poises) comprise entre 950 et 1 050°C, et une température de dévitrification comprise entre 700 et 780°C.

**7.** Composition selon l'une des revendications 1 à 6, **caractérisée en ce qu'**elle est une des suivantes :

- SiO$_2$ 64,29% ; Na$_2$O 18,12% ; K$_2$O 0,47% ; CaO 6,07% ; MgO 2,97% ; B$_2$O$_3$ 5,95% ; et Al$_2$O$_3$ 1,85% (C$_6$) ;

- SiO$_2$ 64,37% ; Na$_2$O 18,46% ; K$_2$O 0,34% ; CaO 5,68% ; MgO 2,95% ; B$_2$O$_3$ 6,01% ; et Al$_2$O$_3$ 1,95% (C$_7$) ;

- SiO$_2$ 64,28% ; Na$_2$O 19,02% ; K$_2$O 0,41 % ; CaO 5,34% ; MgO 2,72% ; B$_2$O$_3$ 5,91 % ; et Al$_2$O$_3$ 2,11% (C$_8$).

**8.** Composition selon l'une des revendications 1, 3 à 6, **caractérisée en ce qu'**elle est une des suivantes :

- SiO$_2$ 66,3% ; Na$_2$O 17,6% ; K$_2$O 0,52% ; CaO 5,95% ; MgO 2,82% ; B$_2$O$_3$ 4,8% ; et Al$_2$O$_3$ 1,75% (C$_9$) ;

- SiO$_2$ 64,86% ; Na$_2$O 18,06% ; K$_2$O 0,51% ; CaO 5,94% ; MgO 2,94% ; B$_2$O$_3$ 5,52% ; et Al$_2$O$_3$ 2,02% (C$_{10}$) ;

- SiO$_2$ 63,24% ; Na$_2$O 17,94% ; K$_2$O 0,38% ; CaO 6,01% ; MgO 3,02% ; B$_2$O$_3$ 6,74% ; et Al$_2$O$_3$ 2,48% (C$_{11}$) ;

- SiO$_2$ 63,05% ; Na$_2$O 19,41% ; K$_2$O 0,44% ; CaO 6% ; MgO 2,96% ; B$_2$O$_3$ 5,49% ; et Al$_2$O$_3$ 2,51% (C$_{12}$) ;

- SiO$_2$ 62,28% ; Na$_2$O 20,11 % ; K$_2$O 0,41 % ; CaO 5,97% ; MgO 2,76% ; B$_2$O$_3$ 5,41% ; et Al$_2$O$_3$ 2,8% (C$_{13}$).

**9.** Produit de laine de verre, dans lequel sont contenues des fibres de la composition définie dans les revendications 1-8.

Fig 1

Fig 2

Fig 3

13